(19) Europäisches Patentamt / European Patent Office / Office européen des brevets

(11) **EP 4 516 904 A1**

(12) **EUROPEAN PATENT APPLICATION**
published in accordance with Art. 153(4) EPC


(43) Date of publication:
**05.03.2025 Bulletin 2025/10**

(21) Application number: **23795560.4**

(22) Date of filing: **27.04.2023**

(51) International Patent Classification (IPC):
***C12N 15/113*** *(2010.01)* ***A61K 31/713*** *(2006.01)*
***A61P 9/12*** *(2006.01)*

(52) Cooperative Patent Classification (CPC):
**A61K 31/713; A61P 9/12; C12N 15/113**

(86) International application number:
**PCT/CN2023/091259**

(87) International publication number:
**WO 2023/208128 (02.11.2023 Gazette 2023/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.04.2022 CN 202210477929**
**09.09.2022 CN 202211106626**



(71) Applicant: **Basecure Therapeutics**
**Wuxi City, Jiangsu 214091 (CN)**

(72) Inventors:
- **LU, Jianyu**
  **Shanghai 200131 (CN)**
- **CHEN, Shi**
  **Shanghai 200131 (CN)**
- **HU, Lihong**
  **Shanghai 200131 (CN)**
- **DING, Charles Z.**
  **Shanghai 200131 (CN)**
- **HE, Haiying**
  **Shanghai 200131 (CN)**
- **CHEN, Shuhui**
  **Shanghai 200131 (CN)**

(74) Representative: **Elkington and Fife LLP**
**Prospect House**
**8 Pembroke Road**
**Sevenoaks, Kent TN13 1XR (GB)**


Remarks:
The complete document including Reference Table(s) and the Sequence Listing(s) can be downloaded from the EPO website

(54) **CONJUGATE OF NUCLEOTIDE ANALOG-CONTAINING DOUBLE-STRANDED RNAI ANALOG**


(57) Disclosed is a conjugate of a nucleotide analog-containing double-stranded RNAi analog, and specifically disclosed are a sequence composition and a ligand structure of a conjugate of the double-stranded RNAi analog.





Processed by Luminess, 75001 PARIS (FR)

## Description

This patent application claims the following priorities:

CN202210477929.8, April 29, 2022;
CN202211106626.1, September 09, 2022.

## TECHNICAL FIELD

The present invention relates to a conjugate of a nucleotide analog-containing double-stranded RNAi analog, in particular to the conjugate or a pharmaceutically acceptable salt thereof and use in a drug for treatment of related diseases.

## BACKGROUND

The RNA interference (RNAi) mechanism has been gaining close attentions since it was discovered in 1998; the mechanism achieves the control of a corresponding target function by downregulating the level of target mRNA so as to achieve expected pharmacological effects. An RNAi drug is generally a ribonucleic acid having a double-stranded structure, which binds to Ago2 protein and the like in target cells after entering the cells via delivery to form an RNA-induced silencing complex (RISC). The complex binds to target mRNA in the target cells, and hydrolyzes and cleaves target the target mRNA so that the target mRNA losses a protein translation function, thereby downregulating the quantity of target proteins to achieve the inhibition of the target function. In recent years, multiple RNAi drugs have been approved on the market and completed drug feasibility verification for application to a human body. Drugs achieving pharmacological effects through the mechanism are known as RNAi drugs.

The renin-angiotensin-aldosterone (RAAS) system plays an important role in blood pressure regulation. The angiotensinogen (AGT) protein is the only known upstream substance in the RAAS system. The AGT protein in a circulatory system is mainly from liver synthesis. The rennin secreted by kidney transforms AGT in the circulatory system into angiotensin I (Ang I), then an angiotensin converting enzyme (ACE) transforms inactive Ang I into angiotensin II (Ang II) having biological activity. Ang II acts on its receptor to take effects of vasoconstriction and promotion of aldosterone release and the like, causing an increase in blood pressure. The regulatory effect of the RAAS system on blood pressure has been deeply verified in clinic, and renin inhibitors (aliskiren), angiotensin-converting inhibitors (drugs with names containing suffix -pril) and angiotensin II receptor antagonists (drugs with names containing suffix -sartan) which target this pathway have become commonly used drugs in clinical treatment of hypertension.

Hypertension is a clinical syndrome mainly characterized by an increased systemic circulation arterial pressure. The continuous increase in blood pressure and blood pressure fluctuation are main risk factors for cardiovascular diseases, and can lead to the damage of target organs such as heart, kidney, brain and blood vessels.

There are a large number of hypertensive patients all over the world. The first comprehensive analysis report on global hypertension trends, authored by Imperial College London and the World Health Organization and published in The Lancet on August 25, 2021, is the largest study on hypertension in the world to date. (Article source: 10.1016/S0140-6736 (21) 01330-1.). This report showed that in the past 30 years, the number of adults aged 30-79 with hypertension has increased from 650 million to 1.28 billion. The study also found that over half of hypertensive patients (53% female and 62% male, totaling 720 million people) did not receive necessary treatment. The hypertension can cause huge harm, and cardiovascular diseases (CVD) caused by hypertension leads to the death of approximately 8.5 million people worldwide every year. Therefore, there is a serious unmet clinical need for effective blood pressure control.

Drugs for clinical treatment of hypertension are main small molecule drugs. The small molecule drugs are prone to causing nocturnal hypertension fluctuation and poor 24-hour blood pressure control due to quick absorption and metabolism, short half-life and insufficient acting time. Furthermore, the small molecule drugs have a high burden of medication and need to be taken daily. In clinical practice, they are prone to missed doses, causing an increase in blood pressure and damage to target organs. Therefore, there is an urgent need for a long-acting antihypertensive drug in clinical practice to fill unmet clinical needs.

## SUMMARY

A first aspect of the present invention provides a conjugate of a double-stranded RNAi analog or a pharmaceutically acceptable salt thereof, wherein the double-stranded RNAi analog comprises a sense strand and an antisense strand capable of forming a double-stranded region,

the sense strand is 5'-as,us,Cf,Cf,a,Cf,Af,Af,u,g,a,g,a,g,u,a,c,a-3' (SEQ ID NO:1), and
the antisense strand is 3'-a,gs,cs,a,g,u,a,Gf,g,Uf,g,u,u,a,c,u,c,Z,c,a,u,Gfs,us,A,C,A,C,U,C-5' (SEQ ID NO:2),

wherein,
Z is

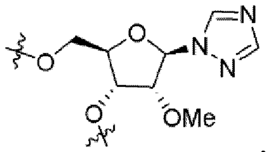

,

the 5' end and/or 3' end of the sense strand and/or antisense strand is conjugated to a ligand, and the ligand is one or more GalNAc derivatives attached through a divalent, trivalent or tetravalent branched linker.

In some embodiments of the first aspect of the present invention, the ligand is one or more GalNAc derivatives attached through a divalent or trivalent branched linker.
In some embodiments of the first aspect of the present invention, the ligand is one or more GalNAc derivatives attached through a trivalent branched linker.
In some embodiments of the first aspect of the present invention, the ligand is a GalNAc derivative attached through a trivalent branched linker.
A second aspect of the present invention provides a conjugate of a double-stranded RNAi analog or a pharmaceutically acceptable salt thereof, wherein the double-stranded RNAi analog comprises a sense strand and an antisense strand capable of forming a double-stranded region,

the sense strand is 5'-as,us,Cf,Cf,a,Cf,Af,Af,u,g,a,g,a,Gf,u,a,c,a-3' (SEQ ID NO:3), and
the antisense strand is 3'-a,gs,cs,a,g,u,a,Gf,g,Uf,g,u,u,a,c,u,c,Z,c,a,u,Gfs,us,A,C,A,C,U,C-5' (SEQ ID NO:2),
wherein,
wherein Z is

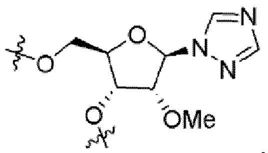

,

the 5' end and/or 3' end of the sense strand and/or antisense strand is conjugated to a ligand, and the ligand is one or more GalNAc derivatives attached through a divalent, trivalent or tetravalent branched linker.

In some embodiments of the second aspect of the present invention, the ligand is one or more GalNAc derivatives attached through a divalent or trivalent branched linker.
In some embodiments of the second aspect of the present invention, the ligand is one or more GalNAc derivatives attached through a trivalent branched linker.
In some embodiments of the second aspect of the present invention, the ligand is a GalNAc derivative attached through a trivalent branched linker.
A third aspect of the present invention provides a conjugate of a double-stranded RNAi analog or a pharmaceutically acceptable salt thereof, wherein the double-stranded RNAi analog comprises a sense strand and an antisense strand capable of forming a double-stranded region,

the sense strand is 5'-as,us,Cf,Cf,a,Cf,Af,Af,u,g,a,Gf,a,g,u,a,c,a-3' (SEQ ID NO:4), and
the antisense strand is 3'-a,gs,cs,a,g,u,a,Gf,g,Uf,g,u,u,a,c,u,c,Z,c,a,u,Gfs,us,A,C,A,C,U,C-5' (SEQ ID NO: 2),
wherein,
Z is

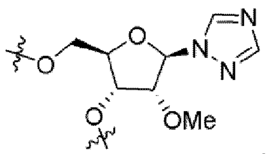

,

the 5' end and/or 3' end of the sense strand and/or antisense strand is conjugated to a ligand, and the ligand is one or more GalNAc derivatives attached through a divalent, trivalent or tetravalent branched linker.

In some embodiments of the third aspect of the present invention, the ligand is one or more GalNAc derivatives attached through a divalent or trivalent branched linker.

In some embodiments of the third aspect of the present invention, the ligand is one or more GalNAc derivatives attached through a trivalent branched linker.

In some embodiments of the third aspect of the present invention, the ligand is a GalNAc derivative attached through a trivalent branched linker.

In some technical embodiments of the present invention, the ligand is conjugated to the 3' end of the sense strand, and other variables are as defined in the present invention.

In some technical embodiments of the present invention, the ligand is conjugated to the 5' end of the sense strand, and other variables are as defined in the present invention.

In some technical embodiments of the present invention, the ligand is conjugated to the 3' end of the antisense strand, and other variables are as defined in the present invention.

In some technical embodiments of the present invention, the ligand is conjugated to the 5' end of the antisense strand, and other variables are as defined in the present invention.

In some technical embodiments of the first, second and/or third aspect of the present invention, the ligand is conjugated to the 3' end of the sense strand, and other variables are as defined in the present invention.

In some technical embodiments of the first, second and/or third aspect of the present invention, the ligand is conjugated to the 5' end of the sense strand, and other variables are as defined in the present invention.

In some technical embodiments of the first, second and/or third aspect of the present invention, the ligand is conjugated to the 3' end of the antisense strand, and other variables are as defined in the present invention.

In some technical embodiments of the first, second and/or third aspect of the present invention, the ligand is conjugated to the 5' end of the antisense strand, and other variables are as defined in the present invention.

A fourth aspect of the present invention provides the following conjugates or pharmaceutically acceptable salts thereof,

Conjugate 1:

sense strand: 5'-as,us,Cf, Cf,a,Cf, Af,Af,u,g,a,g,a,g,u,a,c,a,D01 (SEQ ID NO:5)
antisense strand: 3'-a,gs,cs,a,g,u,a,Gf,g,Uf,g,u,u,a,c,u,c,Z,c,a,u,Gfs,us,A,C,A,C,U,C-5' (SEQ ID NO:2);

Conjugate 2:

sense strand: 5'-as,us,Cf,Cf,a,Cf,Af,Af,u,g,a,g,a,Gf,u,a,c,a,D01 (SEQ ID NO:6)
antisense strand: 3'-a,gs,cs,a,g,u,a,Gf,g,Uf,g,u,u,a,c,u,c,Z,c,a,u,Gfs,us,A,C,A,C,U,C-5' (SEQ ID NO:2);

Conjugate 3:

sense strand: 5'-as,us,Cf,Cf,a,Cf,Af,Af,u,g,a,Gf,a,g,u,a,c,a,D01 (SEQ ID NO:7)
antisense strand: 3'-a,gs,cs,a,g,u,a,Gf,g,Uf,g,u,u,a,c,u,c,Z,c,a,u,Gfs,us,A,C,A,C,U,C-5' (SEQ ID NO:2);
wherein the structure of the D01 is as follows:

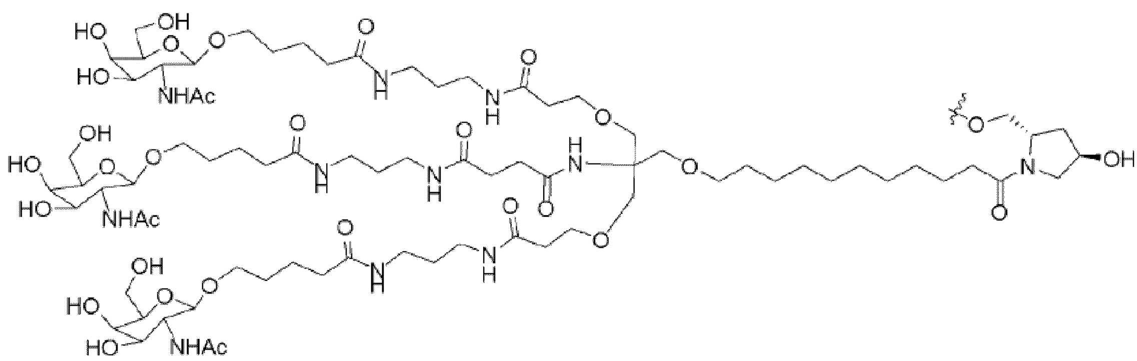

the D01 is linked to a via a phosphodiester bond.

A fifth aspect of the present invention provides a pharmaceutical composition, which comprises an effective dose of a conjugate defined in any technical embodiment mentioned above, or a pharmaceutically acceptable salt thereof and a pharmaceutically acceptable carrier.

In some embodiments of the fifth aspect of the present invention, the pharmaceutical composition comprises an effective dose of any one, two, three, four or five conjugates of double-stranded RNAi analogs mentioned above, or pharmaceutically acceptable salts thereof and a pharmaceutically acceptable carrier.

In some embodiments of the fifth aspect of the present invention, the pharmaceutical composition comprises an effective dose of any one conjugate of double-stranded RNAi analog mentioned above, or a pharmaceutically acceptable salt thereof and a pharmaceutically acceptable carrier.

A sixth aspect of the present invention provides use of any conjugate of double-stranded RNAi analog mentioned above, or the pharmaceutically acceptable salt thereof in preparing a drug for treatment of an AGT-related disease.

In some embodiments of the sixth aspect of the present invention, the AGT-related disease mentioned above is hypertension.

In some embodiments of the sixth aspect of the present invention, the AGT-related disease mentioned above is a cardiovascular disease.

A seventh aspect of the present invention provides a method for treating an AGT-related disease in a subject in need thereof, comprising administrating to the subject an effective dose of conjugate as defined in any technical embodiments mentioned above, or a pharmaceutically acceptable salt thereof or a pharmaceutical composition.

When the information in the sequence listing of the present invention is inconsistent with the specification, the specification shall control.

The present invention also provides test methods below:

**Test method 1: Free uptake experiment of primary human hepatocyte (PHH)**

1. Experimental principle:

A sample to be tested is incubated with primary human hepatocytes. The downregulation degree of AGT mRNA by the sample to be tested is evaluated.

2. Experimental materials:

Primary human hepatocytes (PHH), RNeasy® 96 Kit (12) (QIAGEN-74182), FastKing RT Kit (With gDNase) (Tiangen- KR116-02), TaqMan Gene Expression Assay (GAPDH, Thermo, Assay ID-Hs02786624_g1), TaqMan Gene Expression Assay (AGT, Thermo, Assay ID-Hs01586213_m1).

3. Experimental method:

A conjugate of the present invention is diluted with a PBS solution until the obtained concentration is 10 times a concentration to be tested. 10 μL of siRNA is transferred to a 96-well plate. PHH cells are thawed and seeded to the 96-well plate, and the final cell density is 5.4 x $10^5$ cells/well. The conjugate of the present invention is tested for 10 concentration points, and the highest concentration is 500 nM, with 4-fold dilution.

The cells are incubated for 48 h under the conditions of 37°C and 5% $CO_2$, and the state of the cells is observed using a microscope.

After the incubation is completed, the cells are lysed, all of RNA is extracted from the obtained lysate using RNeasy® 96 Kit (QIAGEN-74182), and underwent reverse transcription using FastKing RT Kit (With gDNase) (Tiangen-KR116-02) to obtain cDNA. AGT cDNA is detected by qPCR.

4. Experimental results: the conjugate of the present invention can significantly downregulate the level of AGT mRNA in PHH cells.

**Test method 2: Mouse hAGT HDI model**

1. Experimental principle:
The targeting of a target gene in a sample to be tested and an inhibitory effect on the target gene are evaluated by a mouse model injected with pcDNA-CMV-AGT plasmids via tail vein at high pressure.
2. Experimental materials: pcDNA-CMV-AGT plasmids, BALB/c female mice, DPBS (Dulbecco's phosphate buffer solution), and a conjugate of the present invention or a pharmaceutically acceptable salt thereof.

3. Experimental method:
6 to 8-week-old BALB/c female mice are purchased, and the mice are adapted to quarantine for one week after arriving at an animal room.

On Day 0, the mice are divided into groups at random according to weight data, with 4 mice in each group, and all the mice are dosed subcutaneously after grouping, with a single dose at 10 mL/kg. The mice in group 1 are dosed with DPBS; the mice in group 2 are dosed with the conjugate of the present invention or the pharmaceutically acceptable salt thereof at 3 mg/kg.

On Day 3 after administration, all the mice are injected with the pcDNA-CMV-AGT plasmids in a volume of 8% of their weight via the tail vein within 5s (injection volume (mL)=mouse weight (g) x 8%). The mass of the plasmids injected into each mouse is 10 μg.

On Day 4 after administration, all groups of mice are euthanized by $CO_2$ inhalation, and 2 liver samples are collected from each mouse. The liver samples are treated with RNAlater at 4°C overnight, then RNAlater is removed, and subsequently the samples without RNAlater are stored at -80°C for detecting the expression level of AGT genes.

4. Experimental conclusion:

The conjugate of the present invention has an excellent downregulation effect on the level of AGT mRNA in mouse liver.

**Test method 3: In vivo PD experiment of non-human primate cynomolgus monkey**

1. Experimental principle:
The downregulation degree of cynomolgus monkey plasma AGT by the sample to be tested is observed through a non-human primate cynomolgus monkey model highly homologous to a human in AGT gene.
2. Experimental materials: cynomolgus monkeys, 1 x PBS (phosphate buffer solution), AD-85481 (US2021095290), and a conjugate of the present invention.
3. Experimental method:
10 cynomolgus monkeys with the weight of 2-3 kg are purchased, and the animals are adapted to quarantine for one week after arriving at an animal room.

On Day -6, the cynomolgus monkeys are divided into groups at random according to weight data, with 2 cynomolgus monkeys in each group. The AGT protein concentrations in plasmas of all the cynomolgus monkeys are detected after grouping as an experimental data baseline for each group.

Administration is performed on Day 1. Blood is collected on Day 1, 2, 4, 8, 15, 22, 29, 32, 35, 43, 57, 71 after administration to detect the AGT protein concentration in plasma of each cynomolgus monkey. By comparing the AGT protein concentration in plasma after administration to the AGT protein concentration on Day -6, the relative downregulation proportion of the AGT protein in each group of animals is obtained.

4. Experimental results: the conjugate of the present invention can significantly downregulate the AGT protein concentration in the plasma of the cynomolgus monkeys.

**Test method 4: In vitro RNA sequencing study**

1. Experiment introduction:

Transcriptome refers to a sum of all RNA transcripted from specific tissues or cells at a certain time or state, mainly including mRNA and non-encoding RNA. Transcriptome sequencing is to study all mRNA transcripted from specific tissues or cells during a certain period based on an Illumina sequencing platform, is a basis for gene functions and structure study, and plays an important role in understanding the development of organisms and the occurrence of diseases. With the development of gene sequencing technologies and reduction in sequencing cost, RNA-seq, with its advantages of high throughput, high sensitivity and wide application range, has become a main method for transcriptome study. The RNA-seq technical process mainly includes two parts: library construction and sequencing, and biological information analysis.

2. RNA extraction and detection:

RNA is extracted from tissues or cells using a standard extraction method, and subsequently serious quality

control is performed on RNA samples. The quality control method is mainly achieved by Agilent 2100 bioanalyzer to precisely detect the integrity of RNA.

3. Library construction and quality inspection:

mRNA is obtained mainly by the following two methods: one is to utilize the structural feature that most mRNA in eukaryotes has polyA tails to enrich mRNA with polyA tails using Oligo (dT) magnetic beads; and the other one is to remove ribosomal RNA from total RNA to obtain mRNA. Subsequently, the obtained mRNA is randomly fragmented using divalent cations in NEB Fragmentation Buffer, and library construction is carried out using either an NEB ordinary library construction method or a strand specific library construction method.

NEB ordinary library construction: a first strand of a cDNA is synthesized in an M-MuLV reverse transcriptase system by using fragmented mRNA as a template and random oligonucleotides as primers. Subsequently, the RNA strand is digested using RNaseH, and a second strand of the cDNA is synthesized in a DNA polymerase I system using dNTPs as a raw material. The purified double-stranded cDNA is subjected to end repair, A tail addition and sequencing linker linkage. About 250-300 bp cDNAs are screened using AMPure XP beads and subjected to PCR amplification, and the PCR product is purified again using AMPure XP beads to finally obtain a library. A kit for the library construction is NEBNext® Ultra™ RNA Library Prep Kit for Illumina®.

Strand specific library construction: a method for synthesizing the first strand of cDNA via reverse transcription is the same as the NEB ordinary library construction method except that when the second strand is synthesized, dTTP in dNTPs is replaced by dUTP, and then end repair, A tail addition, sequencing linker linkage and length screening of cDNA are similarly carried out. Subsequently, the second strand of cDNA containing U is digested using USER enzyme followed by PCR amplification to obtain a library. A strand specific library has many advantages, for example, more effective information can be obtained under the same quantity of data; more accurate gene quantification, localization and annotation information can be obtained; and antisense transcripts and the expression level of a single exon in each isoform can be provided. A kit used for the library construction is NEBNext® Ultra™ Directional RNA Library Prep Kit for Illumina®.

Note: a sequencing linker comprises three components, namely, P5/P7, index and Rd1/Rd2 SP. Among them, P5/P7 is a portion which a PCR amplification primer and a primer on a flow cell bind to, index provides information distinguishing different libraries, Rd1/Rd2 SP, namely read1/read2 sequence primer, is a region which a sequencing primer binds to. The sequencing process theoretically starts from Rd1/Rd2 SP backwards.

After library construction is completed, preliminary quantification is carried out using Qubit2.0 Fluorometer. The library is diluted to 1.5 ng/ul, and subsequently the insert size of the library is detected using Agilent 2100 bioanalyzer. After the insert size meets the expectation, the effective concentration of the library is accurately quantified by qRT-PCR (the effective concentration of the library is higher than 2 nM) to ensure the quality of the library.

4. On-machine sequencing:

After library inspection is qualified, different libraries are subjected to Illumina sequencing after pooling according to effective concentration and target offline data volume requirements. The basic principle of sequencing is sequencing by synthesis. Four fluorescent labeled dNTPs and DNA polymerases as well as linker primers are added in the sequenced flow cell for amplification. When complementary strands are extended in each sequencing cluster, the addition of a fluorescent labeled dNTP can release corresponding fluorescence, and a sequencer captures a fluorescence signal and transforms the light signal into a sequencing peak through computer software to obtain sequence information of a fragment to be tested.

5. Experimental results: the conjugate of the present invention significantly downregulates the AGT gene in RNA-Sequencing study.

**Test method 5: Agonistic activity test of conjugate on hTLR3, hTLR7 and hTLR8 cells**

1. Experimental materials

Table 1 Experimental materials

| Main reagent | Supplier | Article number |
|---|---|---|
| QUANTI-Blue Solution | InvivoGen | rep-qbs2 |
| CellTiter-Glo | Promega | G7573 |

(continued)

| Main reagent | Supplier | Article number |
|---|---|---|
| HEK BLUE hTLR3 | InvivoGen | hkb-htlr3 |
| HEK BLUE hTLR7 | InvivoGen | hkb-htlr7 |
| HEK BLUE hTLR8 | InvivoGen | hkb-htlr8 |
| BLOCK-iT™ Alexa Fluor® Red Fluorescent Control | InvivoGen | 14750100 |
| OPTI MEM | InvivoGen | 11058021 |
| RNAiMAX | InvivoGen | 13778-150 |

2. Experimental Instruments

The main instruments used in this study are multifunctional microplate reader Flexstation III (Molecular Device) and Echo555 (Labcyte).

3. Experimental steps

1) A compound is diluted to a concentration that is 20 times a final concentration, the diluted compound is mixed with RNAiMAX-OPTI MEM (RNAiMAX:OPTI MEM=3 :47) at a ratio of 1:1 and incubated for 15 min at room temperature. ("compound" refers to the conjugate of the present invention for test.)
2) 10 μL of incubated compound is added into a corresponding cell plate, with 10 concentrations in total and double duplicated wells for each concentration.
3) 10 μL of PBS is added in each of negative control wells, 10 μL of 20 μg/mL Poly (I:C) HMW is added into each of hTLR3 positive control wells, and 10 μL of 10 μg/mL R848 is added into each of hTLR7 and hTLR8 positive control wells. 10 μL of 0.5 μM BLOCK-iT™ Alexa Fluor® Red Fluorescent Control is added into 2 transfection positive control wells.
4) 90 μL of cells are seeded into a 96-well plate where the compound has been added with the density of 50,000 cells/well.
5) The compound and the cells are co-incubated for 24 h in an incubator under the conditions of 37°C and 5% $CO_2$.
6) The transfection effect of the transfection positive control wells is observed using a fluorescence microscope, and photographed.
7) Compound activity detection: 20 μL of cell supernatant is taken from each well and added into an experimental plate containing 180 μL of QUANTI-Blue™ reagent and then incubated for 1 h at 37°C, followed by detecting absorbance values at 650nm (OD650) using the multifunctional microplate reader Flexstation III.
8) Cell activity detection: the operation is carried out according to the method in the Celltiter-Glo specification, and chemiluminescence signals (RLU) are detected using the multifunctional microplate reader Flexstation III.

3. Data analysis

Compound activity: the OD650 values are analyzed using GraphPad Prism software, and a dose effect curve of the compound is fit, and the $EC_{50}$ value of the compound is calculated. Cell activity detection: the calculation formula of cell activity % is as follows. The cell activity % value is analyzed using GraphPadPrism software, the dose effect curve of the compound is fit, and the $CC_{50}$ value of the compound on cell is calculated.

$$\text{Cell activity \%=RLU compound/RLUDMSO control*100\%}$$

4. Experimental results:

The conjugate of the present invention exhibits good effects in this test.

**Technical effects**

The conjugate of the present invention is low in off target risk and low in immunogenic risk, and can significantly

downregulate the level of AGT mRNA in PHH cells, the level of mouse liver AGT mRNA in mouse hAGT HDI model, and the AGT protein concentration in non-human primate cynomolgus monkey plasma.

**Definition and illustration**

Unless otherwise specified, the following terms and phrases used herein are intended to have the following meanings. A specific term or phrase should not be considered uncertain or unclear without a specific definition, but should be understood according to the meaning understood by persons of ordinary skill in the art. When a commercial name appears herein, it is intended to refer to its corresponding commercial product or its active ingredient.

Unless otherwise specified, the term "comprising, including and containing" or its equivalent is an open-ended expression, meaning that in addition to the listed elements, components or steps, it may also encompass other unspecified elements, components or steps.

The double-stranded RNAi analog refers to a ribonucleic acid molecule complex which has a double-stranded structure, comprises two reversely parallel and substantially complementary nucleic acid strands, and has "sense" and "antisense" orientations relative to target RNA. In the present disclosure, "complementary" has the meaning well-known by those skilled in the art, namely, in a double-stranded nucleic acid molecule, a base on one strand is paired with a base on the other strand in a complementary manner. Purine base adenine (A) is always paired with pyrimidine base uracil (U); purine base guanine (C) is always paired with pyrimidine base cytosine (G). Each base pair includes one purine and one pyrimidine. When adenine on one strand is always paired with uracil on the other strand and guanine is always paired with cytosine, the two strands are considered as being complementary with each other, and the sequence of the strand can be inferred from the sequence of its complementary strand.

The "modifications" of nucleotide described in the present disclosure include but are not limited to methoxy modification, fluoro modification, (E)-vinyl phosphate modification, thiophosphate linkage, or replacement of nucleotide with GNA (glycerol nucleic acid) nucleotide monomer, and the GNA comprises GNA-A, GNA-T, GNA-C, GNA-G, and GNA-U. The sequences described in the present disclosure can comprise "further modified sequences" in Table 2 below.

The fluoro-modified nucleotide in the present disclosure refers to a nucleotide formed by replacing hydroxyl at the 2' position of the ribosyl group of a nucleotide with fluorine, and the nucleotide subjected to methoxy modification refers to a nucleotide formed by replacing the 2'-hydroxyl of the ribosyl with methoxy.

The thiophosphate linkage methods described in the present disclosure all refer to reading from the 5'end, where the previous nucleotide and the next nucleotide are linked by a thiophosphate bond; for example, 5'-as,us,Cf...-3' of the sense strand, wherein "as,us" represents the linkage of a nucleotide residue a and a nucleotide residue u by the thiophosphate bond, "us,Cf" represents the linkage of a nucleotide residue u and a nucleotide residue Cf by the thiophosphate bond; for example, 3'-a,gs,cs,a...u,Gfs,us,A...-5' of the antisense strand, wherein "Gfs,us" represents linkage of a nucleotide residue u and a nucleotide residue Gf by the thiophosphate bond, "u,Gfs" represents the linkage of a nucleotide residue Gf and a nucleotide residue u by the thiophosphate bond, "gs,cs" represents the linkage of a nucleotide residue c and a nucleotide residue g by the thiophosphate bond, and "a,gs" represents the linkage of a nucleotide residue g and a nucleotide residue a by the thiophosphate bond.

The GNA (glycerol nucleic acid) nucleotide monomers (GNA-A, GNA-T, GNA-C, GNA-G and GNA-U) of the present disclosure:

the structure of GNA-A is

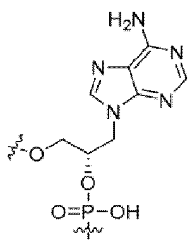

;

the structure of GNA-T is

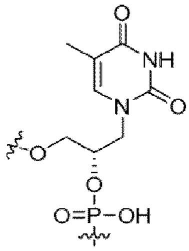

;

the structure of GNA-C is

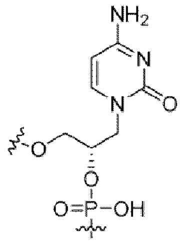

;

the structure of GNA-G is

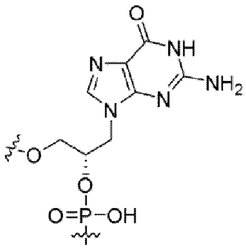

;

and the structure of GNA-U is

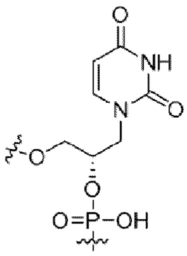

.

The term "multiple" described in the present disclosure refers to an integer greater than or equal to 2, including but not limited to 2, 3, 4, 5, 6, 7, 8, 9, 10, 11, 12, 13, 14, 15, 16, 17, 18, 19, or 20, up to the theoretical upper limit of the siRNA analog.

Unless otherwise specified, the "overhang" described in the present invention refers to at least one unpaired nucleotide protruding from the double-stranded region structure of the double-stranded compound. For example, the 3' end of one strand extends beyond the 5' end of the other strand, or the 5' end of one strand extends beyond the 3' end of the other strand. The overhang can comprise at least one nucleotide; or the overhang can comprise at least two nucleotides, at least three nucleotides, at least four nucleotides, at least five nucleotides or more nucleotides. The nucleotide of the nucleotide overhang is an optional modified nucleotide. The overhang can be located on the sense strand, the antisense strand or any combination thereof. In addition, the overhang can be present in the 5' end or 3' end of the antisense or sense strand of the double-stranded compound, or can be simultaneously present in the two ends. In some embodiments of the present invention, the antisense strand has the overhangs of 1 to 10 nucleotides (for example, 1, 2, 3, 4, 5, 6, 7, 8, 9 or 10 nucleotides) at the 3' end and/or 5' end. In some embodiments of the present invention, the sense strand has the overhangs of 1 to 10 nucleotides (for example, 1, 2, 3, 4, 5, 6, 7, 8, 9 or 10 nucleotides) at the 3' end and/or 5' end. In some

embodiments of the present invention, the antisense strand and the sense strand have the overhangs of 1 to 10 nucleotides (for example, 1, 2, 3, 4, 5, 6, 7, 8, 9 or 10 nucleotides) at the 3' end. In some embodiments of the present invention, the antisense strand and the sense strand have the overhangs of 1 to 10 nucleotides (for example, 1, 2, 3, 4, 5, 6, 7, 8, 9 or 10 nucleotides) at the 5' end.

In the present disclosure, the conjugate of the double-stranded RNAi analog (also known as "conjugate") is a compound formed by linking the double-stranded RNAi analog with a pharmaceutically acceptable conjugation group, and the double-stranded RNAi analog is covalently linked with the pharmaceutically acceptable conjugation group.

In the present disclosure, the pharmaceutically acceptable conjugation group can be linked to the 3' end and/or 5' end of the sense strand and/or antisense strand of the double-stranded RNAi analog.

In some embodiments of the present disclosure, the pharmaceutically acceptable conjugation group is D01.

In the present disclosure, the number of the pharmaceutically acceptable conjugation groups is 1, 2, 3, 4, or 5, and the pharmaceutically acceptable conjugation groups are each independently linked to the 3' end and/or 5' end of the sense strand and/or antisense of the double-stranded RNAi.

In the context of the present disclosure, unless otherwise specified, "conjugation" refers to mutual linkage between two or more chemical moieties each having specific functions in a covalent linkage manner; correspondingly, "conjugate" refers to a compound formed by covalent linkage between the chemical moieties.

In the present disclosure, unless otherwise specified, "linker" refers to an organic moiety group that links two moieties of a compound, such as covalently attaching two moieties of a compound. The linker generally comprises one direct linkage or atom (such as oxygen or sulfur), an atomic group (such as NRR, C(O), C(O)NH, SO, $SO_2$, $SO_2NH$), a substituted or unsubstituted alkyl group, a substituted or unsubstituted alkenyl group, a substituted or unsubstituted alkynyl group, a substituted or unsubstituted aryl group, a substituted or unsubstituted heteroaryl group, a substituted or unsubstituted cycloalkyl group, and a substituted or unsubstituted heterocycloalkyl group, wherein optional one or more C atoms in the substituted or unsubstituted alkyl group, the substituted or unsubstituted alkenyl group and the substituted or unsubstituted alkynyl group can be replaced with the substituted or unsubstituted aryl group, the substituted or unsubstituted heteroaryl group, the substituted or unsubstituted cycloalkyl group and the substituted or unsubstituted heterocycloalkyl group.

A cleavable linker has sufficient stability outside the cell, but it will be cleaved when entering a target cell so as to release the groups of the two moieties fixed jointly by the linker.

The compound of the present disclosure may have a specific geometric or stereoisomeric form. The present disclosure contemplates all such the compounds, including (R)- and (S)-enantiomers, diastereomers and racemic mixtures thereof as well as other mixtures, such as mixtures enriched in enantiomers or diastereomers. All of these mixtures are included within the scope of the present disclosure. Other asymmetric carbon atoms may be present in substituents such as the alkyl group. All of these isomers and their mixtures are all included within the scope of the present disclosure.

Unless otherwise specified, the term "enantiomers" or "optical isomers" refers to stereoisomers that are in mirroring relationship with each other.

Unless otherwise specified, the term "diastereomers" refers to stereoisomers which have two or more chiral centers and are in a non-mirroring relationship.

Unless otherwise specified, a wedge-shaped solid line bond (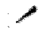) and a wedge-shaped dashed line bond (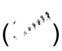) are used to represent an absolute configuration of a stereocenter; a straight solid line bond (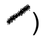) and a straight dashed line bond (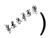) are used to represent a relative configuration of the stereocenter; a wavy line (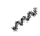) is used to represent the wedge-shaped solid line bond (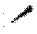) or the wedge-shaped dashed line bond (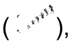), or the wavy line (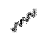) is used to represent the straight solid line bond (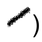) or the straight dashed line bond (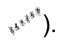).

Unless otherwise specified, the term "enrich in one isomer", "isomer enrichment", "enrich in one enantiomer" or "enantiomer enrichment" refers to a situation that the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomer excess" refers to a difference between relative percentages of two isomers or two enantiomers. For example, if the content of one of the isomers or enantiomers is 90% and the content of the other isomer or enantiomer is 10%, then the excess (ee value) of the isomer or enantiomer is 80%.

Optically active (*R*)- and (*S*)-isomers as well as *D* and *L* isomers can be prepared through chiral synthesis, chiral reagents, or other conventional techniques. If one enantiomer of the compound disclosed herein is desired, it can be prepared by asymmetric synthesis or by derivatization with chiral adjuvants, wherein the resulting diastereomer mixture is

separated and an auxiliary group is cleaved to provide the pure desired enantiomer. Alternatively, when a molecule contains alkaline functional groups (such as amino group) or acidic functional groups (such as carboxyl group), the molecule is reacted with appropriate optically active acids or bases to form salts of diastereomers, and then diastereomer resolution is performed using conventional methods known in the art, followed by recovery to obtain pure enantiomers. In addition, the separation of enantiomers and diastereomers is typically achieved through chromatography, wherein the chromatography adopts a chiral stationary phase and optionally combines with a chemical derivatization method (for example, carbamate is generated from amines). The compound of the present disclosure can comprise non-natural proportions of atomic isotopes on one or more atoms that constitute the compound. For example, the compound can be labeled with a radioactive isotope, such as tritium ($^3H$), iodine-125 ($^{125}I$), or C-14 ($^{14}C$). As another example, hydrogen is replaced with deuterium to form a deuterated drug. A bond formed by deuterium and carbon is stronger than that formed by ordinary hydrogen and carbon. Compared to a non-deuterated drug, the deuterated drug has the advantages of reducing toxic side effects, increasing drug stability, enhancing efficacy, and prolonging drug half-life. All isotopic composition transformations of the compound of the present disclosure, whether radioactive or not, are included within the scope of the present disclosure.

The term "salt" refers to a salt of the compound of the present disclosure, which is prepared from a compound with a specific substituent found in the present disclosure and a relatively non-toxic acid or base. When the compound of the present disclosure comprises relatively acidic functional groups, a base addition salt can be obtained by contacting such compound with a sufficient amount of bases in a pure solution or a suitable inert solvent. The pharmaceutically acceptable base addition salts comprise sodium, potassium, calcium, ammonium, organic amines or magnesium salts, or similar salts. When the compound of the present disclosure comprises relatively basic functional groups, an acid addition salt can be obtained by contacting a sufficient amount of acid with such compound in a pure solution or a suitable inert solvent. Examples of the pharmaceutically acceptable acid addition salts include salts of inorganic acids comprising for example hydrochloric acid, hydrobromic acid, nitric acid, carbonic acid, bicarbonate, phosphoric acid, phosphate monohydrate, dihydrogen phosphate, sulfuric acid, hydrogen sulfate, hydroiodic acid, phosphorous acid, etc; salts of organic acids comprising acetic acid, propionic acid, isobutyric acid, maleic acid, malonic acid, benzoic acid, succinic acid, octanedioic acid, fumaric acid, lactic acid, mandelic acid, phthalic acid, benzenesulfonic acid, p-toluenesulfonic acid, citric acid, tartaric acid, methanesulfonic acid, and other similar acids; salts of amino acids (such as arginine); as well as salts of organic acids such as glucuronic acid. Some specific compounds of the present disclosure contain basic and acidic functional groups, and therefore can be converted into any base or acid addition salt.

The salts of the present disclosure can be synthesized by using parent compounds containing acid or base groups through conventional chemical methods. In general, the preparation method of such the salts is as follows: in water or an organic solvent or their mixture, the salts can be prepared by reacting these compounds in a free acid or base form with an appropriate stoichiometric base or acid.

The compound of the present disclosure can be prepared by multiple synthetic methods well-known to those skilled in the art, including specific embodiments listed below, embodiments formed by combining with other chemical synthesis methods, and equivalent substitution methods well-known to those skilled in the art. Preferred embodiments include but are not limited to the Examples of the present disclosure.

The compound of the present invention can comprise non-natural proportions of atomic isotopes on one or more atoms that constitute the compound. For example, the compounds can be labeled with radioactive isotopes, such as tritium ($^3H$), iodine-125 ($^{125}I$), or C14 ($^{14}C$). As another example, hydrogen can be replaced with deuterium to form a deuterated drug. A bond formed by deuterium and carbon is stronger than the bond formed by ordinary hydrogen and carbon. Compared to a non-deuterated drug, the deuterated drug has the advantages of reducing toxic side effects, increasing drug stability, enhancing efficacy, prolonging drug half-life and the like. All isotopic composition transformations of the compounds of the present invention, whether radioactive or not, are included within the scope of the present invention.

When linkage direction of the listed linkage groups are not indicated, the linkage direction is arbitrary, for example, the linkage group L in

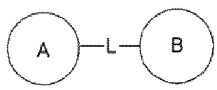

is -M-W-, at this point, -M-W- can link to ring A and ring B in a reading sequence from left to right in the same direction to constitute

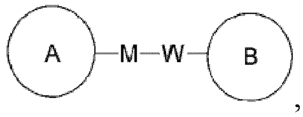

,

or -M-W- can also link to ring A and ring B in a reading sequence from left to right in an opposite direction to constitute

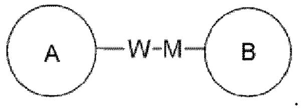

The combinations of the linkage groups, substituents and/or their variants are only allowed if such a combination produces a stable compound.

The term "optional" or "optionally" refers to the possible, but not necessary, occurrence of a subsequent event or situation being described, and the description includes the circumstances in which the event or situation occurs and the circumstances in which the event or situation does not occur.

The term "substituted" refers to any one or more hydrogen atoms on a specific atom being replaced by a substituent. The substituent can include variants of deuterium and hydrogen, as long as the valence state of the specific atom is normal and the substituted compound is stable. When the substituent is oxygen (i.e., =O), it means that two hydrogen atoms are substituted. Oxygen substitution does not occur on aromatic groups. The term "optionally substituted" refers to the situation where a group is substituted or unsubstituted, unless otherwise specified, and the type and number of the substituents can be arbitrary based on chemical feasibility.

When any variable (such as R) appears more than once in the composition or structure of the compound, its definition in each case is independent. Therefore, for example, if a group is substituted by 0-2 R, the group can be optionally substituted by up to two R, and R in each case has independent options. In addition, the substituents and/or their variants can only be allowed as long as the stable compound is produced in such combinations.

When the number of linkage groups is 0, such as $-(CRR)_0-$, it indicates that the linkage group is a single bond.

When one substituent is vacant, it indicates that the substituent is absent. For example, when X in A-X is vacant, it indicates that the structure is actually A. When atoms through which the listed substituents are linked to the substituted group are not indicated, such substituents can be bonded through any atom. For example, a pyridyl group, as a substituent, can be linked to a substituted group through any carbon atom on the pyridine ring.

The compound of the present invention can be prepared by various synthetic methods well-known to those skilled in the art, including specific embodiments listed below, embodiments formed by combining with other chemical synthesis methods, and equivalent substitution methods well-known to those skilled in the art. Preferred embodiments include but are not limited to the Examples of the present invention.

The structure of the compound of the present invention can be confirmed by conventional methods well known to those skilled in the art. If the present invention involves the absolute configuration of the compound, the absolute configuration can be confirmed by conventional technical means in the art. For example, in a single crystal X-ray diffraction (SXRD) method, diffraction intensity data of cultured single crystals is collected using a Bruker D8 venture diffractometer; the light source is CuKα radiation, and the scanning method is φ/ω scanning; and after relevant data are collected, the crystal structure can be further resolved using a direct method (Shelxs97) so as to confirm the absolute configuration.

Solvents used in the present disclosure are commercially available.

Unless otherwise specified, solvent ratios used for column chromatography and preparative thin-layer silica gel chromatography in the present disclosure are all volume ratios.

The present invention adopts the following abbreviations: aq represents aqueous; HATU represents O-(7-azabenzotriazole-1-yl)-N,N,N',N'-tetramethylurea hexafluorophosphate; EDC represents N-(3-dimethylaminopropyl)-N'-ethylcarbodiimide hydrochloride; m-CPBA represents 3-chloroperoxybenzoic acid; eq represents an equivalent or an equal quantity; CDI represents carbonyldiimidazole; DCM represents dichloromethane; PE represents petroleum ether; DIAD represents diisopropyl azodicarboxylate; DMF represents N,N-dimethylformamide; DMSO represents dimethyl sulfoxide; EtOAc represents ethyl acetate; EtOH represents ethanol; MeOH represents methanol; CBz represents benzyloxycarbonyl which is an amine protecting group; BOC represents tert-butoxycarbonyl which is an amine protecting group; HOAc represents acetic acid; $NaCNBH_3$ represents sodium cyanoborocyanide; r.t. represents room temperature; O/N represents overnight; THF represents tetrahydropyran; $Boc_2O$ represents di-tert-butyl dicarbonate; TFA represents trifluoroacetic acid; DLPEA represents diisopropylethylamine; $SOCl_2$ represents thionyl chloride; $CS_2$ represents carbon disulfide; TsOH represents p-toluenesulfonic acid; NFSI represents N-fluoro-N-(benzenesulfonyl) benzenesulfonamide; NCS represents 1-chloropyrrolidine-2,5-dione; *n*-$Bu_4NF$ represents tetrabutylamine fluoride; iPrOH represents 2-propanol; mp represents a melting point; and LDA represents lithium diisopropylamide.

The abbreviations for nucleotide monomers are used in the description of the nucleic acid sequence, specifically as shown in Table 2.

Table 2 Abbreviations for nucleotide monomers

| Abbreviation | Nucleotide |
|---|---|
| A | Adenosine-3'-phosphate |
| U | Uridine-3'-phosphate |
| G | Guanosine -3'- phosphate |
| C | Cytidine-3'- phosphate |
| gs | 2'- O-methylguanosine-3'-thiophosphate |
| us | 2'- O-methyluridine-3'-thiophosphate |
| as | 2'-O-methyladenosine-3'-thiophosphate |
| cs | 2'-O-methylcytidine-3'-thiophosphate |
| c | 2'-O-methylcytidine-3 ' -phosphate |
| a | 2'-O-methyladenosine-3'-phosphate |
| u | 2'-O-methyluridine-3'-phosphate |
| g | 2'-O-methylguanosine-3'-phosphate |
| Cf | 2'-fluorocytidine-3'-phosphate |
| Af | 2'-fluoroadenosine-3'-phosphate |
| Uf | 2'-fluorouridine-3'-phosphate |
| Gf | 2'-fluoroguanosine-3'-phosphate |
| Cfs | 2'-fluorocytidine-3 '-thiophosphate |
| Afs | 2'-fluoroadenosine-3'-thiophosphate |
| Ufs | 2' -fluorouridine-3' -thiophosphate |
| Gfs | 2'-fluoroguanosine-3'-thiophosphate |
| GNA-C | Cytidine glycerol nucleic acid |
| GNA-A | Adenosine glycerol nucleic acid |
| GNA-T | Thymidine glycerol nucleic acid |
| GNA-G | Guanosine glycerol nucleic acid |
| GNA-U | Uridine glycerol nucleic acid |

The compounds are named according to conventional naming principle in the art or using ChemDraw® software, and names provided by Suppliers Directory are adopted for commercially available compounds.

**DETAILED DESCRIPTION**

Next, the present invention will be described in detail through Examples, but does not imply any adverse limitations on the present invention. The compound of the present invention can be prepared by various synthetic methods well-known to those skilled in the art, including specific embodiments listed below, embodiments formed by combining with other chemical synthesis methods, and equivalent substitution methods well-known to those skilled in the art. Preferred embodiments include but are not limited to the Examples of the present invention. It will be apparent to those skilled in the art to make various changes and improvements to the specific embodiments of the present invention without departing from the spirit and scope of the present invention.

**Synthesis example 1: Synthesis of Z1 and Z2**

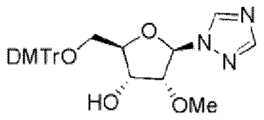

Z1

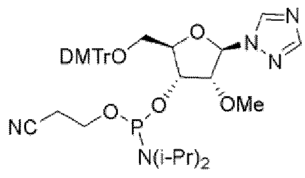

Z2

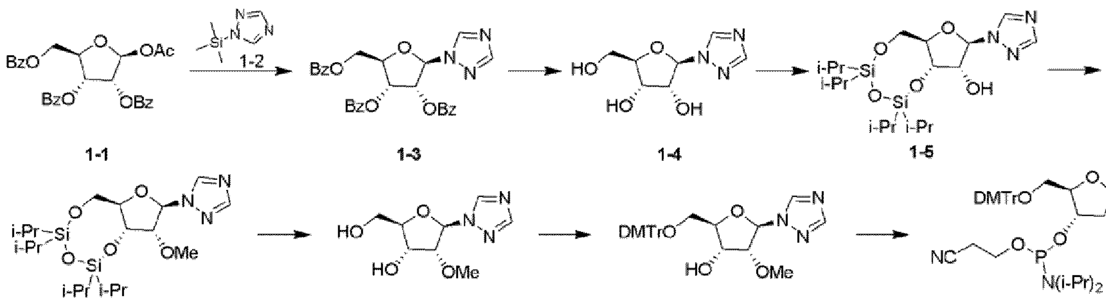


1-1 1-3 1-4 1-5

1-6 1-7 Z1 Z2

Step A: Compound 1-1 (10 g, 19.82 mmol) was dissolved into acetonitrile (120 mL) and 1,2-dichloroethane (80 mL), compound 1-2 (6.02 g, 42.62 mmol) and trimethylsilyl trifluoromethanesulfonate (11.01 g, 49.56 mmol, 8.95 mL) were added into the above mixture at 0°C. The above system was stirred for 12 h at 35°C. The stirred system was quenched by slowly adding a saturated sodium bicarbonate aqueous solution (100 mL) and extracted with dichloromethane (100 mL x 2) at 0°C, and an organic phase was washed with a saturated sodium chloride aqueous solution (200 mL), dried over anhydrous sodium sulfate, filtered and concentrated under reduced pressure to obtain a crude product. The crude product was purified by silica gel column (eluent: petroleum ether/ethyl acetate=20:1) to obtain compound 1-3.

Step B: Compound 1-3 (6.7 g, 13.05 mmol) was dissolved into an ammonia methanol solution (7 mol/L, 50 mL). The above system was stirred for 12 h at 40°C, and the reaction solution was concentrated under reduced pressure to obtain a crude product. The crude product was purified by silica gel column (eluent: ethyl acetate/methanol=50/1 to 10/1) to obtain compound **1-4.**

Step C: Compound **1-4** (2.3 g, 11.43 mmol) was dissolved into anhydrous pyridine (25 mL), 1,3-dichloro-1,1,3,3-tetraisopropyldisiloxane (3.64 g, 11.55 mmol, 3.69 mL) was added at 0°C, and then the above system was stirred for 12 h at 20°C. The stirred system was quenched with water (30 mL) and extracted with ethyl acetate (30 mL x 2). An organic phase was washed successively with hydrochloric acid (1 mol/L, 30 mL x3) and saturated saline (30 mL), dried over anhydrous sodium sulfate, filtered and concentrated under reduced pressure to obtain a crude product. The crude product was purified by silica gel column (eluent, petroleum ether/ethyl acetate=7/1 to 5/1) to obtain compound **1-5.**

- Step D: Compound **1-5** (4 g, 9.02 mmol) was dissolved into acetonitrile (40 mL), and silver oxide (8.36 g, 36.06 mmol), 4A molecular sieve (3 g), anhydrous pyridine (1.78 g, 22.54 mmol, 1.82 mL), and iodomethane (6.4 g, 45.08 mmol, 2.81 mL) were sequentially added. The above system was stirred for 12 h at 25°C. Ethyl acetate (50 mL) was added into the system, the above materials were stirred for 1 h at 20°C and filtered using a Buchner funnel with diatomaceous earth, and the filtrate was collected. The filtrate was extracted with water (100 mL) and ethyl acetate (100 mL x 2). An organic phase was washed with saturated saline (200 mL), dried over anhydrous sodium sulfate, filtered and concentrated under reduced pressure to obtain a crude product. The crude product was purified by silica gel column (eluent, petroleum ether/ethyl acetate=15/1 to 10/1 to 8/1) to obtain compound **1-6.**

Step E: Compound **1-6** (2 g, 4.37 mmol) was dissolved into anhydrous tetrahydrofuran (20 mL), and then triethylamine trihydrofluoride (1.55 g, 9.61 mmol, 1.57 mL) was added. The above system was stirred for 12 h at 20°C, and the reaction solution was concentrated under reduced pressure to obtain a crude product. The crude product was purified by silica gel column (eluent: ethyl acetate/methanol=100/1 to 50/1) to obtain compound **1-7.**

Step F: Compound **1-7** (1 g, 4.65 mmol) was dissolved into anhydrous pyridine (10 mL), and then 4,4-dimethoxytriphenylmethyl chloride (1.57 g, 4.65 mmol) was added. The above system was stirred for 12 h at 20°C. The stirred system was quenched with water (20 mL) and extracted with ethyl acetate (20 mL x 2). An organic phase was washed with saturated saline (40 mL), dried over anhydrous sodium sulfate, filtered and concentrated under reduced pressure to obtain a crude product. The crude product was purified by silica gel column (eluent: petroleum ether/ethyl acetate=3/1 to 1/1) to obtain Z1. $^1H$ NMR (400 MHz, DMSO-$d_6$): $\delta$ = 8.78 (s,1H), 8.14 - 8.03 (m, 1H), 7.41 - 7.31 (m, 2H), 7.29 - 7.16 (m, 7H), 6.92 - 6.76 (m, 4H), 6.08 (d, *J*=3.2Hz, 1H), 5.25 (d, *J* = 6.5 Hz, 1H), 4.48 - 4.34 (m, 1H), 4.13 - 4.01 (m,

2H), 3.73 (s, 6H), 3.38 (s, 3H), 3.18 - 3.03 (m,2H).

Step G: 5 g of raw material Z1 (9.66 mmol, 1.0 eq) was dissolved into 50mL of dichloromethane, 3.2 g of bis(diisopropylamino) (2-cyanoethoxy) phosphine (10.63 mmol, 1.1 eq) and 0.57 g of 4,5-dicyanoimidazole (4.83 mmol, 0.5 eq) were added, and the above materials reacted for 2 h at 25°C. After the reaction was completed, the reaction was quenched with 20 mL of saturated sodium bicarbonate at 20-25°C, diluted with 20 mL of water and extracted with dichloromethane (50 mL x 2), organic phases were combined, washed with saturated sodium chloride (30 mL x 2), dried over anhydrous sodium sulfate and filtered, and then the mother liquor under was concentrated under reduced pressure to obtain a crude product. The crude product was purified by silica gel column (eluent, petroleum ether/ethyl acetate (0.1% triethylamine)=1/0 to 1/1) to obtain 6.8 g of white solid powder **Z2.** 1H NMR (400MHz, DMSO-d6)δ= 8.82 (s,1H), 8.11 (d, J = 6.6Hz,1H),7.42-7.32 (m,2H), 7.30-7.16 (m,7H), 6.90-6.76 (m, 4H), 6.20-6.08 (m, 1H), 4.76-4.51 (m,1H), 4.29 (q,J = 4.2 Hz, 1H), 4.23-4.09 (m, 1H), 3.83-3.75 (m, 1H), 3.72 (d, J = 2.4 Hz, 6H), 3.61 - 3.45 (m, 3H), 3.38 (d,J = 15.2 Hz, 3H), 3.30-3.16 (m, 1H), 3.07 (br dd, J = 5.6, 10.4 Hz, 1H), 2.77 (t, J = 5.9 Hz, 1H), 2.63-2.55 (m,1H), 1.16-1.07 (m, 9H), 0.96 (d, J = 6.6 Hz, 3H).

## Preparation method of conjugate

Preparation method: oligoribonucleotide was synthesized using a phosphoramidite solid-phase synthesis technology. The synthesis was carried out on a solid support made by covalently linking a controllable porous glass (CPG, 500 Å) with compound 2. All RNA phosphoramidites on the sequence were sequentially dissolved into anhydrous acetonitrile, and a molecular sieve (3Å) was added. 5-ethylthio-1H-tetrazole (ETT) was used as an activator, and the coupling time was 5 min. a phosphate bond was generated by using a 50 mM $I_2$-water/pyridine (volume ratio 1/9) solution, with the reaction time being 3 min. A solution of 50 mM 3-((dimethylamino)-methylene) amino)-3H-1,2,4-dithiazole-3-thioketone (DDT) in anhydrous acetonitrile/pyridine (volume ratio of 1/1) was used to generate a thiophosphate bond, with the reaction time being 3 min. The conjugate was synthesized after the DMT group of the obtained sequence was finally removed.

Cleavage and deprotection of an oligomer bound onto CPG: after the solid-phase synthesis was terminated, CPG was treated for 30 min using an acetonitrile solution containing 10% diethylamine to remove a protecting group without cleaving nucleotide from CPG. Subsequently, the dried CPG was treated with a concentrated ammonia solution at 40°C for 18 h. After centrifugation, the supernatant was transferred to a new tube and CPG was washed with water. The combined solution was concentrated to obtain a solid mixture. The solid mixture was dissolved with DMSO (10V), then triethylamine hydrofluride (2V) was added, and the above materials were stirred for 16 h at 40°C. After the reaction was completed, the reaction product was purified.

Purification of oligoribonucleotide: an oligomer was purified using NanoQ anion exchange HPLC. Buffer solution A was a 10 mM sodium perchlorate solution, 20 mM Tris, 1 mM EDTA, pH 7.4 and 20% acetonitrile, as well as buffer solution B was 500 mM sodium perchlorate, 20 mM Tris, 1 mM EDTA, pH 7.4 and 20% acetonitrile. A target product was obtained by separation and desalinated using a reverse phase C18 column.

The oligoribonucleotide was annealed to generate siRNA: a RNA oligomer to be annealed was prepared into a 20 μM RNA oligomer solution using sterile RNase free water (without RNA hydrolase). Equal moles of RNA solutions were combined to form a complementary strand. The annealing reaction system was set as follows: 100 μL of 10 nmol mixed solution was placed in a water bath pot at 95°C for 10 min, then quickly put into water bath at 60°C, and then naturally cooled. The solution after completion of annealing cannot be stored at high temperature.

Examples 1-3 were conducted according to the above methods, the order of raw materials was adjusted based on nucleotide shown in the sequence and the order of Z, so as to prepare the target product.

## Example 1: Preparation of conjugate 1

Sense strand: 5'-as,us,Cf,Cf,a,Cf,Af,Af,u,g,a,g,a,g,u,a,c,a,D01 (SEQ ID NO: 5)
Antisense strand: 3'-a,gs,cs,a,g,u,a,Gf,g,Uf,g,u,u,a,c,u,c,Z,c,a,u,Gfs,us,A,C,A,C,U,C-5' (SEQ ID NO: 2);
Z was

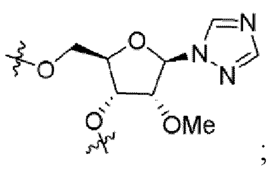

;

wherein the structure of D01 was as follows:

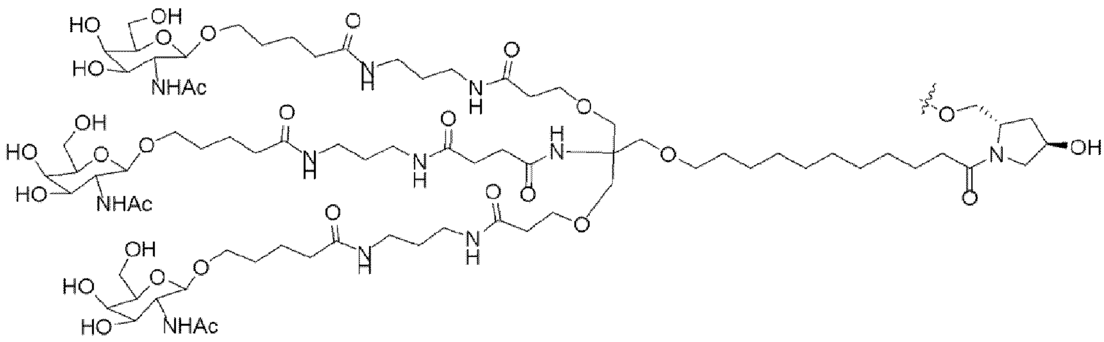

D01 ,

the D01 was linked to a via a phosphodiester bond.

**Example 2: Preparation of conjugate 3**

Sense strand: 5'-as,us,Cf,Cf,a,Cf,Af,Af,u,g,a,Gf,a,g,u,a,c,a,D01 (SEQ ID NO:7)
Antisense strand: 3'-a,gs,cs,a,g,u,a,Gf,g,Uf,g,u,u,a,c,u,c,**Z**,c,a,u,Gfs,us,A,C,A,C,U,C-5' (SEQ ID NO: 2);
Z was

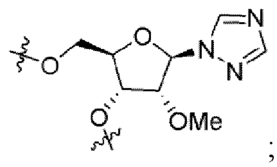

;

wherein the structure of D01 was as follows:

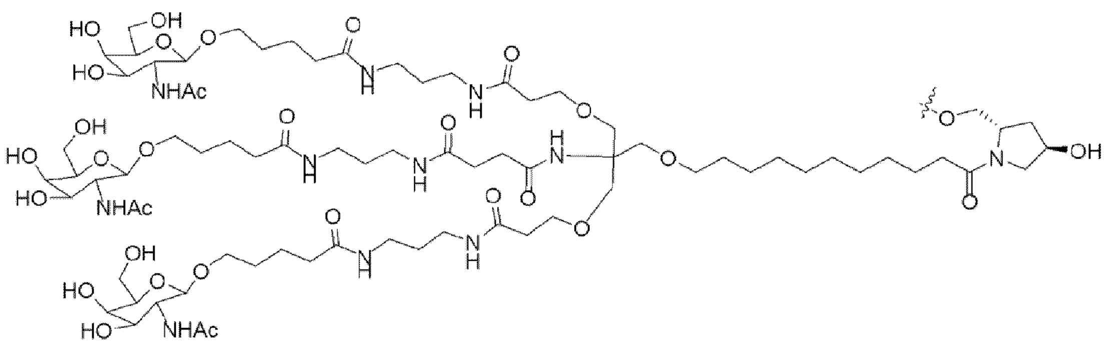

D01 ,

the D01 was linked to a via a phosphodiester bond.

**Example 3: Preparation of conjugate 2**

Sense strand: 5'-as,us,Cf,Cf,a,Cf,Af,Af,u,g,a,g,a,Gf,u,a,c,a,D01 (SEQ ID NO:6)
Antisense strand: 3'-a,gs,cs,a,g,u,a,Gf,g,Uf,g,u,u,a,c,u,c,Z,c,a,u,Gfs,us,A,C,A,C,U,C-5' (SEQ ID NO: 2);
Z was

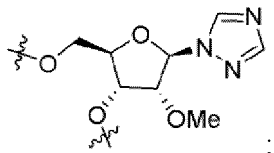

;

wherein the structure of D01 was as follows:

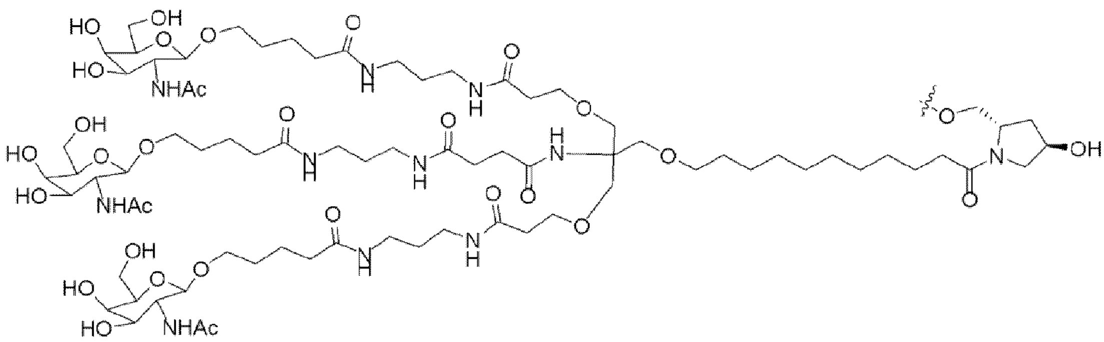

D01

,

the D01 was linked to a via a phosphodiester bond.

## Bioassay

**Test example 1**: **Free uptake experiment of PHH cells**

1. Experimental principle:
A sample to be tested was incubated with primary human hepatocytes. The downregulation degree of AGT mRNA by the sample to be tested was evaluated.
2. Experimental materials:
Primary human hepatocytes (PHH), RNeasy® 96 Kit (12) (QIAGEN-74182), FastKing RT Kit (With gDNase) (Tiangen-KR116-02), TaqMan Gene Expression Assay (GAPDH, Thermo, Assay ID-Hs02786624_g1), TaqMan Gene Expression Assay (AGT, Thermo, Assay ID-Hs01586213_m1).
3. Experimental method:
A conjugate of the present invention was diluted with a PBS solution until the obtained concentration was 10 times the concentration to be tested. 10 uL of siRNA was transferred to a 96-well plate. PHH cells were thawed and seeded to the 96-well plate, and the final cell density was 5.4 x $10^5$ cells/well. The conjugate of the present invention was tested for 10 concentration points, and the highest concentration was 500 nM, with 4-fold dilution.

The cells were incubated for 48 h under the conditions of 37°C and 5% $CO_2$, and the state of the cells was observed using a microscope.

After the incubation was completed, the cells were lysed, all of RNA was extracted from the obtained lysate using RNeasy® 96 Kit (QIAGEN-74182), and underwent reverse transcription using FastKing RT Kit (With gDNase) (Tiangen-KR116-02) to obtain cDNA. AGT cDNA was detected by qPCR.

4. Experimental result: the experimental results are seen in Table 3

Table 3 Free uptake experimental results of conjugate of the present invention by PHH cells

| Cell line | Conjugate number | Log (concentration) | Absolute $EC_{50}$ (nM) | $E_{max}$ (remaining AGT mRNA) | Target gene |
|---|---|---|---|---|---|
| PHH | Conjugate 2 | -0.705 | 0.197 | 5% | AGT |

5. Experimental conclusion: the conjugate of the present invention can significantly downregulate the level of AGT mRNA in PHH cells, and has a good effect of reducing the level of AGT mRNA.

**Test example 2: Mouse hAGT HDI model**

1. Experimental principle:
The targeting of a target gene in a sample to be tested and the inhibitory effect on the target gene were evaluated by a mouse model injected with pcDNA-CMV-AGT plasmids via tail vein at high pressure.
2. Experimental materials:
pcDNA-CMV-AGT plasmids, BALB/c female mice, DPBS (Dulbecco's phosphate buffer solution), and a conjugate of the present invention.
3. Experimental method:
6 to 8-week-old BALB/c female mice were purchased, and the mice were adapted to quarantine for one week after arriving at an animal room.

On Day 0, the mice were divided into groups at random according to weight data, with 4 mice in each group, all the mice were dosed subcutaneously after grouping, with a single dose at 10 mL/kg. The mice in group 1 were dosed with DPBS; the mice in group 2 were dosed with AD-85481 at the dose of 5 mg/kg; the mice in group 3 were dosed with the conjugate 3 of the present invention at the dose of 5 mg/kg; the group 4 was dosed with the conjugate 2 of the present invention at the dose of 5 mg/kg.
On Day 3 after administration, all the mice were injected with the pcDNA-CMV-AGT plasmids at a volume of 8% of their weight via the tail vein within 5s (injection volume (mL)=mouse weight (g) x 8%). The mass of the plasmids injected into each mouse was 10 μg.
On Day 4 after administration, all groups of mice were euthanized by $CO_2$ inhalation, and 2 liver samples were collected from each mouse. The liver samples were treated with RNAlater at 4°C overnight, then RNAlater was removed, and subsequently the samples without RNAlater were stored at -80°C for detecting the expression level of AGT genes.
4. Experimental results: the results are as shown in Table 4. In the table, the data represent AGT mRNA expression of mouse liver after administration/AGT mRNA expression of mouse liver before administration,

Table 4. Downregulation of liver AGT mRNA level in mice by conjugates of the present invention

| Animal group | Group 1 | Group 2 | Group 3 | Group 4 |
|---|---|---|---|---|
| | Phosphate buffer solution, (%) | AD-85481, 5 mg/kg, (%) | Conjugate 3, 5 mg/kg, (%) | Conjugate 2, 5 mg/kg, (%) |
| 1 | 106 | 7 | 2 | 3 |
| 2 | 89 | 9 | 3 | 4 |
| 3 | 94 | 8 | 3 | 2 |
| 4 | 110 | 8 | 2 | 2 |
| Average | 100 | 8 | 3 | 3 |

5. Experimental conclusion: the conjugates of the present invention can significantly downregulate the expression of AGT mRNA in mouse liver, with the downregulation range being up to 97%.

**Test example 3: In vivo PD experiment of non-human primate cynomolgus monkey**

1. Experimental principle:
The downregulation degree of cynomolgus monkey plasma AGT by the sample to be tested was observed through a non-human primate cynomolgus monkey model highly homologous to a human in AGT gene.
2. Experimental materials: cynomolgus monkeys, 1 x PBS (phosphate buffer solution), AD-85481 (US2021095290), and a conjugate of the present invention.
3. Experimental method:

10 cynomolgus monkeys with the weight of 2-3 kg were purchased, and the animals were adapted to quarantine for one week after arriving at an animal room.

On Day -6, cynomolgus monkeys were divided into groups at random according to weight data, with 2 cynomolgus monkeys in each group, the AGT protein concentrations in plasmas of all the cynomolgus monkeys were detected after grouping as an experimental data baseline for each group.

Administration was performed on Day 1. Group 1 was dosed with 1 x PBS; group 2 was subcutaneously injected with AD-85481 at the dose of 0.3 mpk; group 3 was subcutaneously injected with AD-85481 at the dose of 3 mpk; group 4 was subcutaneously injected with the conjugate 2 of the present invention at the dose of 0.3 mpk; group 5 was subcutaneously injected with the conjugate 2 of the present invention at the dose of 3 mpk. Blood was collected on Day 1, 2, 4, 8, 15, 22, 29, 32, 35, 43, 57, 71 after administration to detect the AGT protein concentration in plasma of each cynomolgus monkey. By comparing the AGT protein concentration in plasma after administration to the AGT protein concentration on Day -6, the relative downregulation proportion of the AGT proteins in each group of animals was obtained.

4. Experimental results: the results are shown in Table 5. In the table, the data represent AGT gene expression level after administration/AGT gene expression level before administration.

Table 5. Downregulation of plasma AGT protein level in cynomolgus monkeys after single subcutaneous administration of conjugate of the present invention

| | | Group 1 | Group 2 | Group 3 | Group 4 | Group 5 |
|---|---|---|---|---|---|---|
| | | Phosphate buffer solution (%) | AD-85481 0.3 mg/kg (%) | AD-85481 3 mg/kg (%) | Conjugate 2 0.3 mg/kg (%) | Conjugate 2 3 mg/kg (%) |
| On Day 22 after administration | 1 | 103.12 | 29.25 | 6.18 | 28.47 | 0.84 |
| | 2 | 96.74 | 37.63 | 5.20 | 22.96 | 4.09 |
| | Average | 99.93 | 33.44 | 5.69 | 25.72 | 2.47 |
| On Day 71 after administration | 1 | 109.63 | 65.03 | 8.79 | 55.17 | 10.84 |
| | 2 | 89.94 | 138.97 | 13.22 | 48.35 | 8.92 |
| | Average | 99.79 | 102.00 | 11.00 | 51.76 | 9.88 |

5. Experimental conclusion: the conjugate of the present invention can significantly downregulate the concentration of the AGT proteins in the plasma of the cynomolgus monkey. Compared to the plasma AGT protein concentration on Day -6, the AGT protein concentration on day 22 after single administration for the 3 mpk group was downregulated by 97.53%, and the AGT protein concentration on day 71 was downregulated by 90.12%.

**Test example 4: In vitro RNA sequencing study**

1. Experiment introduction:

Transcriptome refers to a sum of all RNA transcripted from specific tissues or cells at a certain time or state, mainly including mRNA and non-encoding RNA. Transcriptome sequencing is to study all mRNA transcripted from specific tissues or cells during a certain period based on an Illumina sequencing platform, is a basis for gene functions and structure study, and plays an important role in understanding the development of organisms and the occurrence of diseases. With the development of gene sequencing technologies and reduction in sequencing cost, RNA-seq, with its advantages of high throughput, high sensitivity and wide application range, has become a main method for transcriptome study. The RNA-seq technical process mainly includes two parts: library construction and sequencing, and biological information analysis.

2. RNA extraction and detection:

RNA was extracted from tissues or cells using a standard extraction method, and subsequently serious quality control was performed on RNA samples. The quality control method was mainly achieved by Agilent 2100 bioanalyzer to precisely detect the integrity of RNA.

3. Library construction and quality inspection:

mRNA was obtained mainly by the following two methods: one was to utilize the structural feature that most mRNA in eukaryotes had polyA tails to enrich mRNA with polyA tails using Oligo (dT) magnetic beads; and the other one was to remove ribosomal RNA from total RNA to obtain mRNA. Subsequently, the obtained mRNA was randomly fragmented using divalent cations in NEB Fragmentation Buffer, and library construction was carried out using either an NEB ordinary library construction method or a strand specific library construction method.

NEB ordinary library construction: a first strand of a cDNA was synthesized in an M-MuLV reverse transcriptase system by using fragmented mRNA as a template and random oligonucleotides as primers. Subsequently, the RNA strand was digested using RNaseH, and a second strand of the cDNA was synthesized in a DNA polymerase I system using dNTPs as a raw material. The purified double-stranded cDNA was subjected to end repair, A tail addition and sequencing linker linkage. About 250-300 bp cDNAs were screened using AMPure XP beads and subjected to PCR amplification, and the PCR product was purified again using AMPure XP beads to finally obtain a library. A kit for library construction was NEBNext® Ultra™ RNA Library Prep Kit for Illumina®.

Strand specific library construction: a method for synthesizing the first strand of cDNA was the same as the NEB ordinary library construction method except that when the second strand was synthesized, dTTP in dNTPs was replaced by dUTP, and then end repair, A tail addition, sequencing linker linkage and length screening of cDNA were similarly carried out. Subsequently, the second strand of cDNA containing U was digested using USER enzyme followed by PCR amplification to obtain the library. A strand specific library construction has many advantages, for example, more effective information can be obtained under the same quantity of data; more accurate gene quantification, localization and annotation information can be obtained; and antisense transcripts and the expression level of a single exon in each isoform can be provided. A kit used for the library construction was NEB Next® Ultra™ Directional RNA Library Prep Kit for Illumina®.

Note: a sequencing linker comprises three components, namely, P5/P7, index and Rd1/Rd2 SP. Among them, P5/P7 is a portion which a PCR amplification primer and a primer on a flow cell bind to, index provides information distinguishing different libraries, Rd1/Rd2 SP, namely read1/read2 sequence primer, is a region which a sequencing primer binds to. The sequencing process theoretically starts from Rd1/Rd2 SP backwards.

After library construction was completed, preliminary quantification was carried out using Qubit2.0 Fluorometer. The library was diluted to 1.5 ng/uL, and subsequently the insert size of the library was detected using Agilent 2100 bioanalyzer. After the insert size met the expectation, the effective concentration of the library was accurately quantified by QRT-PCR (the effective concentration of the library was higher than 2 nM) to ensure the quality of the library.

4. On-machine sequencing:

After library inspection was qualified, different libraries were subjected to Illumina sequencing after pooling according to effective concentration and target offline data volume requirements. The basic principle of sequencing was sequencing by synthesis. Four fluorescent labeled dNTPs and DNA polymerases as well as linker primers were added in the sequenced flow cell for amplification. When complementary strands were extended in each sequencing cluster, the addition of a fluorescent labeled dNTP can release corresponding fluorescence, and a sequencer captured a fluorescence signal and transformed the light signal into a sequencing peak through computer software to obtain sequence information of a fragment to be tested.

5. Experimental results: the results are as shown in Table 6.

Table 6. In vitro RNA sequencing study results of conjugate of the present invention

| Compound | Downregulate | | | Upregulate | | | Total | | |
|---|---|---|---|---|---|---|---|---|---|
| | N=1 | N=2 | Average | N=1 | N=2 | Average | N=1 | N=2 | Average |
| AD-85481 | 1055 | 1313 | 1184 | 1547 | 713 | 1130 | 2602 | 2026 | 2314 |
| Conjugate 2 | 1006 | 309 | 658 | 1033 | 410 | 722 | 2039 | 719 | 1379 |

6. Experimental conclusion: the conjugate of the present invention has low off target risk.

**Test example 5 Agonistic activity of conjugate of the present invention on hTLR3, hTLR7 and hTLR8 cells**

1. Experimental materials

Table 7 Experimental materials

| Main reagent | Supplier | Article number |
|---|---|---|
| QUANTI-Blue Solution | InvivoGen | rep-qbs2 |
| CellTiter-Glo | Promega | G7573 |
| HEK BLUE hTLR3 | InvivoGen | hkb-htlr3 |
| HEK BLUE hTLR7 | InvivoGen | hkb-htlr7 |
| HEK BLUE hTLR8 | InvivoGen | hkb-htlr8 |
| BLOCK-iT™ Alexa Fluor® Red Fluorescent Control | InvivoGen | 14750100 |
| OPTI MEM | InvivoGen | 11058021 |
| RNAiMAX | InvivoGen | 13778-150 |

2. Experimental instruments

The main instruments used in this study are multifunctional microplate reader Flexstation III (Molecular Device) and Echo555 (Labcyte).

3. Experimental steps

1) The conjugate of the present invention was diluted to a concentration that was 20 times a final concentration, the diluted conjugate was mixed with RNAiMAX-OPTI MEM (RNAiMAX:OPTI MEM=3:47) at a ratio of 1:1 and incubated for 15 min at room temperature.
2) 10 μL of incubated conjugate of the present invention was added into a corresponding cell plate, with 10 concentrations in total, a maximum concentration being 200 nM, and double duplicated wells for each concentration.
3) 10 μL of PBS was added in each of negative control wells, 10 μL of 20 μg/mL Poly (I:C) HMW was added into each of hTLR3 positive control wells, and 10 μL of 10 μg/mL R848 was added into each of hTLR7 and hTLR8 positive control wells. 10 μL of 0.5 μM BLOCK-iT™ Alexa Fluor® Red Fluorescent Control was added into 2 transfection positive control wells.
4) 90 μL of cells were seeded into a 96-well plate with the conjugate at the density of 50,000 cells/well.
5) The conjugate of the present invention and the cells were co-incubated for 24 h in an incubator under the conditions of 37°C and 5% $CO_2$.
6) The transfection effect of the transfection positive control wells was observed using a fluorescence microscope, and photographed.
7) Compound activity detection: 20 μL of cell supernatant was taken from each well and added into an experimental plate containing 180 μL of QUANTI-Blue™ reagent and then incubated for 1 h at 37°C, followed by detecting an absorbance values at 650 nm (OD650) using the multifunctional microplate reader Flexstation III.
8) Cell activity detection: the operation was carried out according to the method in the Celltiter-Glo specification, and a chemiluminescence signals (RLU) were detected using the multifunctional microplate reader Flexstation III.

4. Data analysis

Compound activity: the OD650 values were analyzed using GraphPad Prism software, and a dose effect curve of the compound was fit, and the $EC_{50}$ value of the compound was calculated. Cell activity detection: the calculation formula of cell activity % was as follows. The cell activity % value was analyzed using GraphPadPrism software, the dose effect curve of the compound was fit, and the $CC_{50}$ value of the compound on cell was calculated.

$$\text{Cell activity \%=RLU compound/RLUDMSO control*100\%}$$

5. Experimental results: the experimental results are as shown in Tables 8-10.

Table 8. Agonistic activity of conjugate on hTLR3 cells

| Number | Compound | hTLR3 | | Unit |
|---|---|---|---|---|
| | | $EC_{50}$ | $CC_{50}$ | |
| 1 | AD-85481 | >200 | >200 | nM |
| 2 | Conjugate 3 | >200 | >200 | nM |
| 3 | Conjugate 2 | >200 | >200 | nM |
| 4 | Poly(I:C) HMW | 59.43 | >2000 | ng/mL |

Table 9. Agonistic activity of conjugate on hTLR7 cells

| Number | Compound | hTLR7 | | Unit |
|---|---|---|---|---|
| | | $EC_{50}$ | $CC_{50}$ | |
| 1 | AD-85481 | >200 | >200 | nM |
| 2 | Conjugate 3 | >200 | >200 | nM |
| 3 | Conjugate 2 | >200 | >200 | nM |
| 4 | R848 | 0.200 | >10 | μg/mL |

Table 10. Agonistic activity of conjugate on hTLR8 cells

| Number | Compound | hTLR8 | | Unit |
|---|---|---|---|---|
| | | $EC_{50}$ | $CC_{50}$ | |
| 1 | AD-85481 | >200 | >200 | nM |
| 2 | Conjugate 3 | >200 | >200 | nM |
| 3 | Conjugate 2 | >200 | >200 | nM |
| 4 | R848 | 1.482 | >10 | μg/mL |

5. Experimental conclusion: the conjugates of the present invention do not trigger hTLR3, hTLR7 and hTLR8 responses at the experimental conditions, indicating that the conjugates of the present invention have low immunogenic risk.

**Test example 6. Compound activity experiment in Huh7 cell line**

**1.Objective:**

this study aims to evaluate the inhibitory effect of the conjugate described in this patent on a target gene using a Huh7 cell line. The inhibitory activity of the compound on target gene AGT is evaluated based on the $EC_{50}$ value of the compound as an index.

**2. Experimental materials:**

**2.1 Cell line:** Huh 7 cells, and a conjugate of the present invention

Huh7 cells were provided by Shanghai WuXi AppTec New Drug Development Co., Ltd. Hub7 cells were cultured in MEM medium containing 10% fetal bovine serum and 1% penicillin streptomycin.

**2.2 Reagents:**

The main reagents used in this experiment include a Lipofectamine™iRNAiMAX transfection reagent, FastStart Universal Probe Mast, an RNA extraction kit, a GAPDH gene expression kit, an AGT gene expression kit, a FastKing cDNA First Chain Synthesis Kit, and a 96-well plate.

### 2.3 Consumable material and instruments:

The main instruments used in this experiment include a fluorescence qPCR instrument (Applied Biosystems, model QuantStudio 6 Flex) and a cell counter (Vi-cellTM XR).

### 3. Experimental steps and method:

### 3.1 Transfection

1) On Day 1, a mixed solution of an RNAiMAX transfection reagent and Opti-MEM was prepared as needed in a ratio of RNAiMAX transfection reagent:Opti-MEM=1.5:48.5, and incubated for 15 min at room temperature;
2) For cells in each well, a certain amount of diluted compound was taken and added into an equal amount of RNAiMAX Optim-MEM medium to be mixed well, and then incubated for 15 min;
3) Huh7 cells were taken and washed with DPBS, digested with trypsin, and the density of the cells was adjusted to an appropriate density;
4) The cells were seeded into a 96-well plate at a density of 2,0000 cells/well while adding a mixture of 20 μL Opti-MEM RNAiMax and the compound into the cell culture plate, and the volume of the final medium for each well was 120 μL.
5) The cells were incubated for 24 h in an incubator under the conditions of 5% $CO_2$ and 37°C.

### 3.2 RNA extraction and reverse transcription

The cells were collected, and RNA was extracted according to the instructions of Qiagen-74182 RNA Extraction Kit and then underwent reverse transcription to obtain cDNA according to the instructions of FastKing cDNA First Chain Synthesis Kit.

### 3.3 qPCR detection

The cDNA of the target gene was detected by qPCR. qPCR was carried out in a 384-well plate using GAPDH as an internal reference gene. The qPCR reaction procedure was as follows: heat for 10 min at 95°C and then enter a cycle mode, heat for 15 s at 95°C and for 1 min at 60°C, with 40 cycles in total.

### 3.4 Data analysis: a ΔΔCt method (Ct difference comparison method)

This method requires the introduction of the internal reference gene GAPDH, as the internal reference gene is expressed in all the cells and its product is necessary for maintaining cell survival; and the expression level or copy number of the genome in cells remains constant and is less affected by environmental factors. After qRT-PCR, the CT value of the internal reference will also be recorded, called Ct (GAPDH), and the CT value of the sample is called Ct (sample).

$$\Delta Ct\ (sample) = Ct\ (sample) - Ct\ (GAPDH)$$

$$\Delta Ct\ (control) = Ct\ (control) - Ct\ (GAPDH)$$

$$\Delta\Delta Ct\ (sample) = \Delta Ct\ (sample) - \Delta Ct\ (control)$$

$$\text{The relative expression level of genes} = 2\text{\textasciicircum}\Delta\Delta Ct$$

4. Experimental results: the experimental results are as shown in table 11.

Table 11. Results of transfection experiment of conjugate of the present invention in Huh7 cell

| Cell line | Compound | Log (concentration) | Absolute $EC_{50}$ (pM) | $E_{max}$ (remaining AGT mRNA) | Target gene |
|---|---|---|---|---|---|
| Huh7 | Conjugate 2 | -2.228 | 5.916 | 5% | AGT |

5. Experimental conclusion: the conjugate of the present invention can significantly downregulate the level of AGT mRNA in Huh7 cells, and has a good effect of reducing AGT mRNA.

## Claims

1. A conjugate of a double-stranded RNAi analog or a pharmaceutically acceptable salt thereof, wherein the double-stranded RNAi analog comprises a sense strand and an antisense strand capable of forming a double-stranded region, wherein the sense strand is 5'-as,us,Cf,Cf,a,Cf,Af,Af,u,g,a,g,a,g,u,a,c,a-3' (SEQ ID NO:1), and the antisense strand is 3'-a,gs,cs,a,g,u,a,Gf,g,Uf,g,u,u,a,c,u,c,**Z**,c,a,u,Gfs,us,A,C,A,C,U,C-5' (SEQ ID NO:2),
wherein, Z is

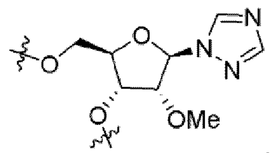

,

the 5' end and/or 3' end of the sense strand and/or antisense strand is conjugated to a ligand, and the ligand is one or more GalNAc derivatives attached through a divalent, trivalent or tetravalent branched linker.

2. A conjugate of a double-stranded RNAi analog or a pharmaceutically acceptable salt thereof, wherein the double-stranded RNAi analog comprises a sense strand and an antisense strand capable of forming a double-stranded region, wherein the sense strand is 5'-as,us,Cf,Cf,a,Cf,Af,Af,u,g,a,g,a,Gf,u,a,c,a-3' (SEQ ID NO:3), and the antisense strand is 3'-a,gs,cs,a,g,u,a,Gf,g,Uf,g,u,u,a,c,u,c,**Z**,c,a,u,Gfs,us,A,C,A,C,U,C-5' (SEQ ID NO:2),
wherein,
Z is

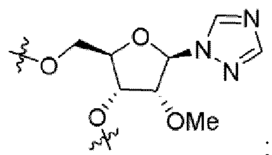

;

the 5' end and/or 3' end of the sense strand and/or antisense strand is conjugated to a ligand, and the ligand is one or more GalNAc derivatives attached through a divalent, trivalent or tetravalent branched linker.

3. A conjugate of a double-stranded RNAi analog or a pharmaceutically acceptable salt thereof, wherein the double-stranded RNAi analog comprises a sense strand and an antisense strand capable of forming a double-stranded region, wherein the sense strand is 5'-as,us,Cf,Cf,a,Cf,Af,Af,u,g,a,Gf,a,g,u,a,c,a-3' (SEQ ID NO:4), and the antisense strand is 3'-a,gs,cs,a,g,u,a,Gf,g,Uf,g,u,u,a,c,u,c,**Z**,c,a,u,Gfs,us,A,C,A,C,U,C-5' (SEQ ID NO: 2),
wherein, Z is

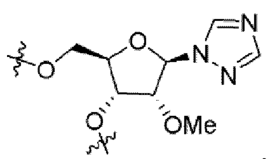

,

the 5' end and/or 3' end of the sense strand and/or antisense strand is conjugated to a ligand, and the ligand is one or more GalNAc derivatives attached through a divalent, trivalent or tetravalent branched linker.

4. The conjugate or pharmaceutically acceptable salt thereof according to any one of claims 1-3, wherein the ligand is conjugated at the 3' end of the sense strand.

5. The conjugate or pharmaceutically acceptable salt thereof according to any one of claims 1-3, wherein the ligand is

conjugated at the 5’ end of the sense strand.

6. The conjugate or pharmaceutically acceptable salt thereof according to any one of claims 1-3, wherein the ligand is conjugated at the 3’ end of the antisense strand.

7. The conjugate or pharmaceutically acceptable salt thereof according to any one of claims 1-3, wherein the ligand is conjugated at the 5’ end of the antisense strand.

8. The conjugate or pharmaceutically acceptable salt thereof according to any one of claims 1-3, wherein the conjugate is selected from:

Conjugate 1:

sense strand: 5’-as,us,Cf, Cf,a,Cf, Af,Af,u,g,a,g,a,g,u,a,c,a,D01 (SEQ ID NO:5)
antisense strand: 3’-a,gs,cs,a,g,u,a,Gf,g,Uf,g,u,u,a,c,u,c,**Z**,c,a,u,Gfs,us,A,C,A,C,U,C-5’ (SEQ ID NO:2);

Conjugate 2:

sense strand: 5’-as,us,Cf,Cf,a,Cf,Af,Af,u,g,a,g,a,Gf,u,a,c,a,D01 (SEQ ID NO:6)
antisense strand: 3’-a,gs,cs,a,g,u,a,Gf,g,Uf,u,u,a,c,u,c,**Z**,c,a,u,Gfs,us,A,C,A,C,U,C-5’ (SEQ ID NO:2);

Conjugate 3:

sense strand: 5’-as,us,Cf,Cf,a,Cf,Af,Af,u,g,a,Gf,a,g,u,a,c,a,D01 (SEQ ID NO:7)
antisense strand: 3’-a,gs,cs,a,g,u,a,Gf,g,Uf,g,u,u,a,c,u,c,**Z**,c,a,u,Gfs,us,A,C,A,C,U,C-5’ (SEQ ID NO:2);
wherein, the structure of the D01 is as follows:

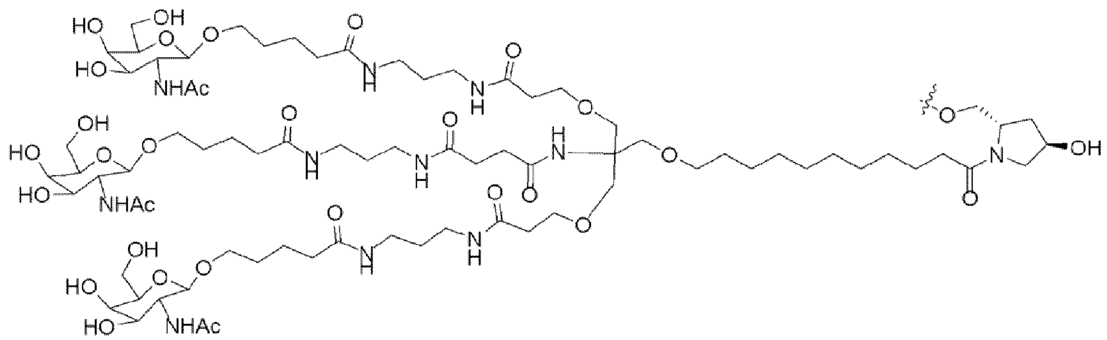

**D01**

the D01 is linked to a via a phosphodiester bond.

**INTERNATIONAL SEARCH REPORT**

International application No.
**PCT/CN2023/091259**

**A. CLASSIFICATION OF SUBJECT MATTER**

C12N15/113(2010.01)i; A61K31/713(2006.01)i; A61P9/12(2006.01)i

According to International Patent Classification (IPC) or to both national classification and IPC

**B. FIELDS SEARCHED**

Minimum documentation searched (classification system followed by classification symbols)

IPC: C12N,A61K,A61P

Documentation searched other than minimum documentation to the extent that such documents are included in the fields searched

Electronic data base consulted during the international search (name of data base and, where practicable, search terms used)

TWMED; TWTXT; USTXT; DWPI; ENTXTC; KRTXT; CNTXT; WPABSC; JPTXT; CNABS; EPTXT; CNMED; WPABS; ENTXT; WOTXT; CJFD; CATXT; VEN, genbank, pubmed, elsevier science, 中国专利生物序列检索系统, Chinese Patent Biological Sequence Retrieval System: 胸苷-乙二醇核酸, 血压, 衍生, AGT, AUCCACAAUGAGAGUACA, ctcacatgtacnctca ttgtggatgacga, derivative, GalNAc, gna, iRNA, RNAi, siRNA, Tgn, AD-85481, SEQ ID NOs: 1-7的序列检索

**C. DOCUMENTS CONSIDERED TO BE RELEVANT**

| Category* | Citation of document, with indication, where appropriate, of the relevant passages | Relevant to claim No. |
|---|---|---|
| A | CN 112852809 A (ALNYLAM PHARMACEUTICALS INC.) 28 May 2021 (2021-05-28)<br>see abstract, and claims 1-5 and 31-45 | 1-8 |
| A | WO 2021249352 A1 (CHIA TAI TIANQING PHARMACEUTICAL GROUP CO., LTD. et al.) 16 December 2021 (2021-12-16)<br>see abstract, claims 1-14, and description, page 12, paragraph 2, and tables 1-3 | 1-8 |
| A | US 2021095290 A1 (ALNYLAM PHARMACEUTICALS INC.) 01 April 2021 (2021-04-01)<br>see abstract, claims 1-30, and description, paragraphs 12 and 21 | 1-8 |
| A | WO 2021110148 A1 (MEDSHINE DISCOVERY INC.) 10 June 2021 (2021-06-10)<br>see abstract, and claims 1-21 | 1-8 |
| A | Maja M. Janas et al. "Selection of GalNAc-conjugated siRNAs with limited off-target-driven rat hepatotoxicity"<br>*Nat Commun.*, Vol. 9, 19 February 2018 (2018-02-19),<br>see entire document | 1-8 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 August 2023** | **24 August 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/091259**

**C. DOCUMENTS CONSIDERED TO BE RELEVANT**

| Category* | Citation of document, with indication, where appropriate, of the relevant passages | Relevant to claim No. |
|---|---|---|
| A | Nair Jayaprakash K et al. "Multivalent N-Acetylgalactosamine-Conjugated siRNA Localizes in Hepatocytes and Elicits Robust RNAi-Mediated Gene Silencing" *Journal of the American Chemical Society,* Vol. 136, No. 49, 10 December 2014 (2014-12-10), 16958-61 see entire document | 1-8 |

Form PCT/ISA/210 (second sheet) (July 2022)

**INTERNATIONAL SEARCH REPORT**

International application No.
**PCT/CN2023/091259**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2023/091259**

| Patent document cited in search report | Publication date (day/month/year) | Patent family member(s) | Publication date (day/month/year) |
|---|---|---|---|
| CN 112852809 A | 28 May 2021 | WO 2015179724 A1 | 26 November 2015 |
| | | WO 2015179724 A8 | 18 February 2016 |
| | | WO 2015179724 A9 | 10 March 2016 |
| | | SG 11201609376 SA | 29 December 2016 |
| | | JP 2017517511 A | 29 June 2017 |
| | | JP 6811094 B2 | 13 January 2021 |
| | | IL 248816 A0 | 31 January 2017 |
| | | IL 248816 B | 29 July 2021 |
| | | US 2023123192 A1 | 20 April 2023 |
| | | BR 122020023687 B1 | 07 March 2023 |
| | | IL 281638 A | 31 May 2021 |
| | | IL 281638 B1 | 01 March 2023 |
| | | IL 281638 B2 | 01 July 2023 |
| | | EP 3739048 A1 | 18 November 2020 |
| | | AU 2015264038 A1 | 01 December 2016 |
| | | AU 2015264038 A2 | 22 December 2016 |
| | | AU 2015264038 B2 | 11 February 2021 |
| | | US 2019298842 A1 | 03 October 2019 |
| | | US 10814007 B2 | 27 October 2020 |
| | | AU 2021202552 A1 | 27 May 2021 |
| | | CA 2948381 A1 | 26 November 2015 |
| | | US 2017189541 A1 | 06 July 2017 |
| | | US 10238749 B2 | 26 March 2019 |
| | | BR 112016026950 A2 | 31 October 2017 |
| | | BR 112016026950 B1 | 07 March 2023 |
| | | KR 20170005130 A | 11 January 2017 |
| | | KR 102410687 B1 | 22 June 2022 |
| | | JP 2022104985 A | 12 July 2022 |
| | | MX 2021006053 A | 06 July 2021 |
| | | KR 20220087576 A | 24 June 2022 |
| | | JP 2021063085 A | 22 April 2021 |
| | | JP 7101748 B2 | 15 July 2022 |
| | | SG 10202104570 TA | 29 June 2021 |
| | | EP 3146049 A1 | 29 March 2017 |
| | | EP 3146049 B1 | 26 February 2020 |
| | | US 2021046187 A1 | 18 February 2021 |
| | | US 11419942 B2 | 23 August 2022 |
| | | EA 201692370 A1 | 31 March 2017 |
| | | MX 2016015126 A | 23 February 2017 |
| | | ZA 201607666 B | 30 January 2019 |
| WO 2021249352 A1 | 16 December 2021 | AU 2021288648 A1 | 09 February 2023 |
| | | AU 2021288648 A8 | 29 June 2023 |
| | | TW 202214855 A | 16 April 2022 |
| | | US 2023235330 A1 | 27 July 2023 |
| US 2021095290 A1 | 01 April 2021 | PE 20210114 A1 | 19 January 2021 |
| | | EP 3794122 A1 | 24 March 2021 |
| | | US 2021310006 A1 | 07 October 2021 |
| | | JP 2021522841 A | 02 September 2021 |
| | | IL 278539 B | 01 June 2022 |
| | | MX 2020012048 A | 29 January 2021 |

Form PCT/ISA/210 (patent family annex) (July 2022)

**INTERNATIONAL SEARCH REPORT**
**Information on patent family members**

International application No.
**PCT/CN2023/091259**

| Patent document cited in search report | Publication date (day/month/year) | Patent family member(s) | Publication date (day/month/year) |
| --- | --- | --- | --- |
| | | IL 292877 A | 01 July 2022 |
| | | CO 2020015314 A2 | 19 March 2021 |
| | | KR 20210010529 A | 27 January 2021 |
| | | WO 2019222166 A1 | 21 November 2019 |
| | | SG 11202011144 QA | 30 December 2020 |
| | | CL 2021002326 A1 | 13 May 2022 |
| | | US 11015201 B2 | 25 May 2021 |
| | | TW 202016304 A | 01 May 2020 |
| | | BR 112020022943 A2 | 17 February 2021 |
| | | CA 3100003 A1 | 21 November 2019 |
| | | AU 2019269418 A1 | 07 January 2021 |
| | | CL 2020002865 A1 | 30 April 2021 |
| | | PH 12020551904 A1 | 21 June 2021 |
| WO 2021110148 A1 | 10 June 2021 | US 2023220386 A1 | 13 July 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- CN 202210477929
- CN 202211106626
- US 2021095290 A

### Non-patent literature cited in the description

- The Lancet on. Imperial College London and the World Health Organization, 25 August 2021